# DEMANDE DE BREVET EUROPEEN

(11) **EP 2 092 951 A1**
(43) Date de publication de la demande: **26.08.2009**
(21) Numéro de dépôt: 08447012.9
(22) Date de dépôt: 20.02.2008
(51) Int. Cl.: A61N 1/04, A61N 1/34, A61N 1/36, A61N 1/32

(54) **Appareil pour le traîtement électrothérapeutique des céphalées de tension**

(71) Demandeur: STX-MED SPRL, 4031 Liege (BE)
(72) Inventeur: Rigaux, Pierre, 4020 Liege (BE); Muller, Pierre-Yves, 1245 Genève (CH)
(74) Mandataire: pronovem

(57) **Abrégé**

La présente invention se rapporte à un appareil pour le traitement électrothérapeutique des céphalées, telles que les céphalées de tension et les migraines, **caractérisé en ce que** :
- le support d'électrode (10) présente une forme et des dimensions choisies pour permettre, de manière indépendante du sujet, l'excitation des afférences des nerfs supratrochléaires (2) et supraorbitaires (3) de la branche ophtalmique (1) du nerf trijumeau ;
- le circuit électrique comprend un générateur de signal programmable apte à créer des impulsions de durée comprise entre 150 et 450 microsecondes avec une montée en intensité de 0 à 20 milliampères maximum à une vitesse inférieure ou égale à 40 microampères par seconde et avec un pas d'augmentation de l'intensité ne dépassant pas 50 microampères.

## Description

### Objet de l'invention

La présente invention se rapporte à un appareil électrique présentant des électrodes appliquées sur la moitié supérieure du visage et visant au traitement des migraines et céphalées.

### Arrière-plan technologique et état de la technique

Une technique d'électrothérapie antalgique connue est la neurostimulation électrique transcutanée, communément appelée TENS (acronyme pour l'appellation anglaise : *Transcutaneous Electrical Nerve Stimulation*). Celle-ci consiste à exciter des afférences sensitives au moyen d'électrodes placées sur la peau, afin de diminuer ou bloquer la douleur (théorie du portillon, stimulation de la production d'analgésiques endogènes). Cette technique est largement connue ^{(1,2,3)} et régulièrement utilisé pour lutter contre divers types de douleurs.

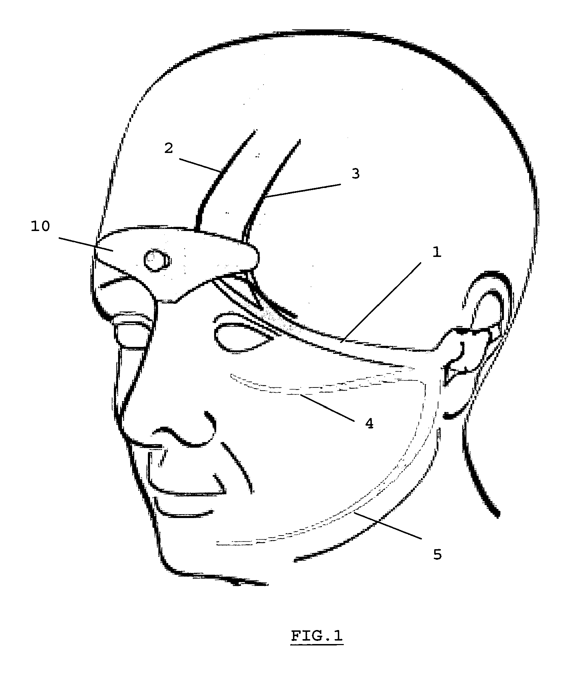

La plupart des migraines et des céphalées de tension se projettent en surface sur la moitié antérieure du crâne. La voie afférente de cette douleur est localisée au niveau du nerf trijumeau (*Trigeminus nervus*), représenté sur la FIG.1, qui comporte trois branches, la branche ophtalmique 1, la branche maxillaire 4 et la branche mandibulaire 5. La voie afférente de la douleur précitée est plus précisément la branche supérieure du nerf trijumeau 1 encore appelé le nerf ophtalmique de Willis.

Ce nerf se divise en deux branches au niveau du front : le nerf frontal interne 2 (ou supratrochléaire) et le nerf frontal externe 3 (ou supraorbitaire).

Ainsi, l'application de l'électrothérapie antalgique de type TENS sur les nerfs supraorbitaire et supratrochléaire permet de lutter contre les douleurs de la plupart des migraines et céphalées de tension comme cela est décrit dans la littérature médicale ^{(4, 5, 6, 7)}.

La demande internationale WO 2006/063417 A1, appartenant à la Demanderesse, décrit un appareil pour l'électro-inhibition des muscles de la face, commercialisé sous le nom *Safetox*^{®} *Beauty,* qui applique des courants électriques via une électrode bipolaire placée au niveau du front. Les deux pôles de l'électrode appartiennent à une structure unique comprenant une pièce de tissu servant de support. Les électrodes sont couvertes d'un gel conducteur autocollant sur leur face interne destinée à être en contact avec la peau. Elles se présentent sous forme de deux zones argentées conductrices qui imprègent le tissu qui est donc également conducteur sur sa face externe en contact avec les patins d'alimentation électrique. Cet appareil s'utilise sans causer de douleur importante. Ceci est possible du fait que l'électrode dudit appareil a été conçue pour être de très petite taille. Typiquement les deux pôles de l'électrode ont une surface maximale comprise entre 100 et 200 mm² et sont séparés par une distance de 4 à 8 mm. La surface de périoste concernée est très faible et le courant pénètre très peu profondément dans les tissus.

Ce type d'électrode, de par sa surface limitée, permet d'agir seulement sur les nerfs supratrochléaires (frontaux internes) mais est sans action sur les nerfs supraorbitaires (frontaux externes) : il ne rencontre donc pas complètement les critères d'efficacité pour un traitement d'électrothérapie antalgique de type TENS dans le cas des migraines et céphalées.

Lorsqu'on utilise une électrode plus large de forme habituelle en vue d'exciter les afférences des nerfs supraorbitaires (frontaux externes), les courants générés par le *Safetox*^{®} *Beauty* provoquent des douleurs qui rendent le traitement précité insupportable.

Le document WO 2006/051370 A1 se rapporte à un dispositif d'électrothérapie appliqué en particulier au traitement des migraines et autres céphalées, névralgies, zonas ophtalmiques, syndrome d'Arnold, etc. Il s'agit d'un dispositif comprenant au moins une électrode pour l'application d'un courant de traitement et un moyen de contrôle. Ce dernier comprend au moins un moyen de déclenchement pour initier la fourniture d'un profil de courant prédéterminé dans ses différents paramètres, notamment l'intensité, et un moyen de stabilisation, qui, lorsqu'il est actionné, provoque la modification dudit profil de courant en limitant l'intensité du courant à sa valeur au moment de l'actionnement dudit moyen de stabilisation. On limiterait ainsi le risque de douleur trop intense pour le patient, lors de l'application d'un profil de courant prédéterminé, grâce au moyen de stabilisation que le patient peut lui-même actionner. Les électrodes sont au nombre de quatre et associées deux à deux latéralement, chaque voie disposant d'une électrode frontale sus-orbitaire et une électrode zygomatique. Cette disposition correspond à la projection en surface de la trifurcation du nerf trijumeau au niveau zygomatique et du passage sur l'arcade sourcilière de sa branche ophtalmique. Le support d'électrode en tissu comprend notamment un bandeau pourvu de deux manchons coulissants permettant d'ajuster la position des électrodes au niveau des arcades sourcilières, ce qui permet de tenir compte des spécificités morphologiques de chaque patient.

### Problème technique à résoudre

L'application d'électrodes et de courant de type TENS au niveau du front pour exciter les afférences des nerfs supraorbitaires et supratrochléaires produit donc des douleurs difficilement supportables. Ces douleurs expliquent pourquoi la technique d'électrothérapie TENS, pourtant très populaire, est très peu, voire pas du tout, utilisée dans le traitement des migraines et des céphalées. En effet, les patients ne peuvent supporter le traitement tant la douleur est vive au niveau du front.

Le périoste qui recouvre la surface de l'os frontal est très richement innervé en fibres nerveuses de la douleur (nociceptives). La proximité entre la surface d'une électrode appliquée sur le front et le périoste de l'os frontal explique le déclenchement de la douleur par les impulsions électriques.

### Buts de l'invention

La présente invention vise à fournir une solution qui permette notamment de résoudre le problème technique précité.

La présente invention a donc pour but de mettre au point une forme d'électrode ainsi que de choisir des paramètres électriques tels que l'application de l'électrothérapie antalgique de type TENS au niveau du front soit efficace mais aussi sans douleur et confortable pour le patient.

La présente invention vise encore à fournir un système d'électrode qui permette de s'adapter de manière simple à la morphologie de l'utilisateur.

### Principaux éléments caractéristiques de l'invention

Un premier objet de la présente invention se rapporte à un appareil pour le traitement électrothérapeutique des céphalées, telles que les céphalées de tension et les migraines, comprenant un élément symétrique allongé, de support de deux électrodes de contact à appliquer transversalement au niveau de la partie supérieure du visage, dans la région inter-sourciliaire, chacune des électrodes étant en contact avec un gel conducteur autocollant appliqué sur la face du support destinée à être appliquée sur la peau du visage, ledit gel conducteur étant appliqué sur deux zones latérales données, couvrant essentiellement tout le support, à l'exception d'une zone centrale isolante, ainsi qu'un circuit électrique pour l'alimentation desdites électrodes au moyen d'impulsions électriques basse tension, **caractérisé en ce que :**
- le support d'électrode présente une forme et des dimensions choisies pour permettre, de manière indépendante du sujet, l'excitation des afférences des nerfs supratrochléaires et supraorbitaires de la branche ophtalmique du nerf trijumeau ;
- le circuit électrique comprend un générateur de signal programmable apte à créer des impulsions de durée comprise entre 150 et 450 microsecondes avec une montée en intensité de 0 à 20 milliampères maximum à une vitesse inférieure ou égale à 40 microampères par seconde et avec un pas d'augmentation de l'intensité ne dépassant pas 50 microampères.

Selon une forme d'exécution préférée de l'invention, le support d'électrode possède une partie centrale de hauteur H1 supérieure à la hauteur H2 de chacune des parties externes, les extrémités supérieures des deux parties externes étant, une fois le support correctement positionné sur le visage, à un niveau légèrement inférieur au niveau de la partie centrale.

Avantageusement, le support d'électrode possède, comme dimensions :
- une longueur ou extension latérale L comprise entre 70 et 115 mm ;
- une hauteur de la partie centrale H1 comprise entre 15 et 50 mm ;
- une hauteur de chacune des parties externes H2 comprise entre 5 et 20 mm.

De manière particulièrement avantageuse,
- la longueur L a une valeur d'environ 95 mm ;
- la hauteur de la partie centrale H1 a une valeur d'environ 30 mm ;
- la hauteur de chacune des parties externes H2 a une valeur d'environ 10 mm.

Toujours avantageusement, la durée des impulsions est d'environ 250 microsecondes.

Toujours avantageusement, le pas d'augmentation de l'intensité est d'environ 30 microampères.

Dans les pays où une telle méthode est brevetable, un deuxième objet de l'invention concerne une méthode de traitement thérapeutique des céphalées, telles que les céphalées de tension et les migraines, au moyen de l'appareil précité, caractérisé au moins par les étapes suivantes :
- on place le support d'électrode autocollant sur la partie supérieure du visage dans la région inter-sourciliaire de manière à couvrir, de chaque côté, les afférences du nerf supratrochléaire et supraorbitaire de la branche ophtalmique du nerf trijumeau ;
- on génère, au moyen du générateur de signal programmable, un signal électrique comprenant des impulsions rectangulaires de durée comprise entre 150 et 450 microsecondes, de préférence 250 microsecondes, avec une montée en intensité de 0 à 20 milliampères maximum, à une vitesse inférieure ou égale à 40 microampères par seconde, ladite montée en intensité étant réalisée avec un pas ne dépassant pas 50 microampères, de préférence 30 microampères ;
- on applique ledit signal électrique aux électrodes pour traiter les céphalées de tension et les migraines.

### Brève description des figures

La FIG.1, déjà mentionnée, représente schématiquement le nerf trijumeau et ses différentes branches, ainsi que le positionnement y relatif de l'électrode selon la présente invention au niveau de la partie supérieure du visage

La FIG.2 représente schématiquement la forme détaillée de l'électrode selon la présente invention.

### Description d'une forme d'exécution préférée de l'invention

On se référera à la demande précitée de la Demanderesse pour ce qui concerne la description générale de l'appareil d'électrothérapie antalgique selon l'invention, qui peut ainsi être considéré comme un perfectionnement de l'appareil décrit dans la demande précitée.

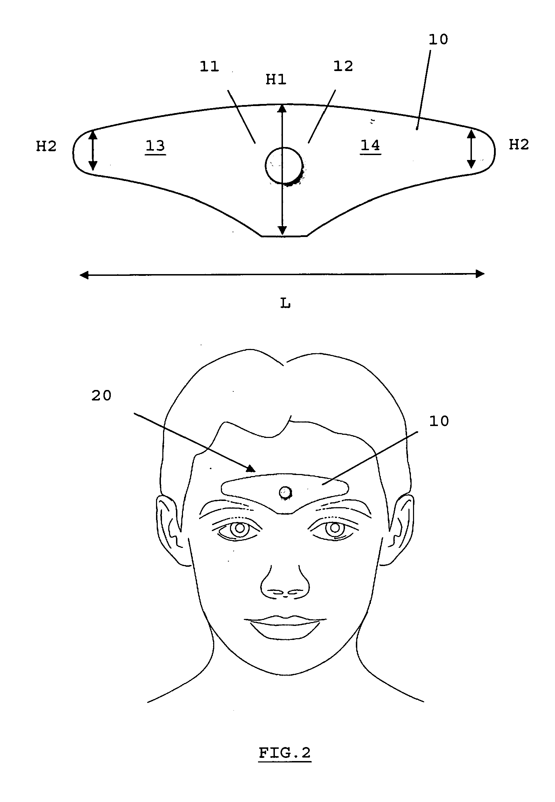

Ainsi, l'appareil selon l'invention, représenté sur la FIG.2, comprend un élément symétrique allongé 10, de support de deux électrodes de contact 11, 12 à appliquer transversalement au niveau de la partie supérieure du visage, dans la région inter-sourciliaire 20, chacune des électrodes 11, 12 étant en contact avec un gel conducteur autocollant appliqué sur la face du support destinée à être appliquée sur la peau du visage. Ledit gel conducteur est appliqué sur deux zones latérales données 13, 14, couvrant essentiellement tout le support 10, à l'exception d'une zone centrale isolante (non représentée). L'appareil comprend également un circuit électrique pour l'alimentation desdites électrodes 11, 12 au moyen d'impulsions électriques basse tension.

Selon l'invention, comme représenté sur la FIG.1, le support d'électrode 10 présente des dimensions choisies pour permettre, de manière indépendante du sujet, l'excitation des afférences des nerfs supratrochléaires 2 et supraorbitaires 3 de la branche ophtalmique 1 du nerf trijumeau.

La présente invention porte plus précisément sur les quatre aspects suivants :
- la forme et les dimensions de l'électrode ;
- la durée de l'impulsion rectangulaire ;
- la progressivité de la montée en intensité et
- le pas de la montée en intensité.

### Caractéristiques de l'électrode

L'électrode selon l'invention est conçue pour à la fois être efficace et limiter la douleur. Pour être efficace, elle doit permettre l'excitation des nerfs supratrochléaires et supraorbitaires droits et gauches chez tous les sujets quelle que soit la circonférence du crâne. Pour limiter la douleur elle doit avoir la plus faible surface possible afin de réduire l'excitation des fibres nerveuses de la douleur (nociceptives).

La forme générale de l'électrode 10 est schématisée sur la FIG.2. Celle-ci est allongée transversalement, symétrique et présente une hauteur centrale supérieure à la hauteur aux deux extrémités de l'électrode.

Les dimensions de l'électrode 10 sont :
- une longueur L comprise entre 70 et 115 mm avec une valeur optimale de 95 mm ;
- une hauteur de la partie centrale H1 comprise entre 15 et 50 mm avec une valeur optimale de 30 mm ;
- une hauteur de la partie externe H2 comprise entre 5 et 20 mm avec une valeur optimale de 10 mm.

Toutes autres forme et/ou dimensions que pourraient concevoir l'homme de métier sont soit inefficaces, car elles ne permettent pas d'exciter les afférences des nerfs-cibles (supraorbitaires et supratrochléaires gauches et droits), soit génératrices de douleurs supplémentaires.

### Caractéristique des impulsions utilisées

Pour exciter des fibres nerveuses, l'électrothérapie antalgique TENS utilise généralement des impulsions rectangulaires. En effet, la forme rectangulaire est décrite dans la littérature comme la plus appropriée (8, 9)

La littérature scientifique décrit que la durée des impulsions rectangulaire doit être égale ou voisine de la chronaxie des fibres nerveuses cibles, c'est-à-dire les fibres nerveuses qui doivent être excitées par l'impulsion. La chronaxie est la durée minimale d'application du courant électrique avec une intensité double de l'intensité minimale (ou *rhéobase*) pour provoquer une excitation. C'est ainsi que l'énergie électrique correspondante fournie aux tissus est minimale ⁽¹⁰⁾. Le matériel haut de gamme du fabriquant principal (CefarCompex) des appareils de TENS utilise d'ailleurs un système de capteur pour mesurer la chronaxie des fibres nerveuses et ensuite régler la durée des impulsions rectangulaires égale à la chronaxie mesurée. Le fait que l'énergie électrique transmise est minimale lorsque la durée de l'impulsion est réglée à une valeur égale à la chronaxie des fibres nerveuses cibles est ainsi décrit dans le manuel d'électrothérapie théorique de CefarCompex ⁽¹¹⁾.

Les fibres nerveuses cibles sont, dans le cas de la présente invention, les afférences sensitives des nerfs supraorbitaires et supratrochléaires. La chronaxie de ces fibres sensitives de type Aβ est comprise entre 30 et 100 µs (microsecondes) avec une moyenne à 50 µs. C'est ainsi que la plupart des appareils d'électrothérapie antalgique TENS utilisent des impulsions rectangulaires de durée comprise entre 30 et 100 µs et généralement d'environ 50 µs.

Les tests de laboratoire de la Demanderesse ont établi que ces durées d'impulsion étaient très douloureuses pour le patient et on a découvert que, contrairement à ce qui était attendu, des impulsions beaucoup plus larges étaient, pour une raison non encore élucidée, beaucoup plus confortables.

Selon la présente invention, les durées d'impulsion adéquates sont alors comprises entre 150 et 450 µs avec un optimum à 250 µs, c'est-à-dire de 3 à 9 fois supérieure aux durées d'impulsions habituellement utilisées dans les appareils d'électrothérapie antalgique TENS selon l'état de la technique.

### Profil de la montée en intensité

Il est connu que l'intensité qui doit être atteinte pour exciter efficacement les afférences sensitives des nerfs supraorbitaires et supratrochléaires se situe entre 8 et 18 mA (milliampères). Le réglage des intensités se fait habituellement avec les appareils de TENS en quelques minutes. Dans la région qui nous intéresse, cette montée en intensité est douloureuse, ce qui rend difficile voire impossible d'arriver à l'intensité efficace de traitement située entre 8 et 18 mA.

Le système nerveux s'habitue à une sensation donnée en modifiant son seuil de perception de la douleur : c'est le mécanisme de tolérance. En tirant parti du phénomène de tolérance, il est possible d'augmenter très progressivement l'intensité sans produire de douleur. Il s'agit de laisser le système nerveux relever son seuil de perception de la douleur avant d'augmenter l'intensité de façon très légère et de manière à rester sous le seuil de la douleur. Puis ainsi de suite à mesure que le système nerveux modifie son seuil de perception de la douleur.

Les tests de laboratoire précités ont permis d'établir que la pente de montée de l'intensité apte à éviter la sensation douloureuse en exploitant le mécanisme de tolérance doit se situer en dessous d'une valeur de 17 mA en 7 minutes, soit une vitesse de montée de l'intensité égale ou inférieure à 40 µA/s (microampères par seconde).

Ceci est vrai pour autant que le pas d'augmentation de l'intensité soit également adapté (voir ci-dessous). Les pentes de montée d'intensité plus raides que celle précitée seront génératrices de douleurs alors que les pentes plus douces seront tolérées.

### Valeur de pas d'augmentation de l'intensité

La montée de l'intensité se fait par pas. Le pas dépend du système électronique générateur des impulsions. Si le pas d'augmentation de l'intensité d'une impulsion à l'autre est trop important, le sujet perçoit cette augmentation brutale de l'intensité et donc une douleur.

Les tests de laboratoire précités ont permis d'établir que la valeur du pas de montée de l'intensité d'une impulsion à l'autre ne doit pas dépasser 50 µA avec un optimum à 30 µA.

Lorsque le pas de montée d'intensité d'une impulsion à l'autre est inférieur à 50 µA, le sujet ne perçoit donc pas d'augmentation brutale d'intensité et ne ressent pas de douleur.

### Bibliographie

**(1)** Melzack, R, Wall, P, Pain mechanisms: A new theory, Science 150: 971-979, 1965;
**(2)** Wall, PD, Sweet, WH, Temporary abolition of pain in man, Science 155: 108-109, 1967;
**(3)** Woolf, CJ, Mitchell, D, Barrett, GD, Antinociceptive effect of peripheral segmental electrical stimulation in the rat, Pain 8: 237-252, 1980.
**(4)** Ahmed HE, White PF, Craig WF, et al., Use of percutaneous electrical nerve stimulation (PENS) in the short-term management of headache, Headache 2000 Apr; 40(4): 311-5.
**(5)** Farina S, Granella F, Malferrari G, Manzoni GC, Headache and cervical spine disorders: classification and treatment with transcutaneous electrical nerve stimulation, Headache 1986 Sep; 26(8): 431-3.
**(6)** Solomon S, Guglielmo KM, Treatment of headache by transcutaneous electrical stimulation, Headache 1985; 25(1): 12-5.
**(7)** Allais G, De Lorenzo C, Quirico PE, Lupi G, Airola G, Mana O, Benedetto C., Non-pharmacological approaches to chronic headaches: transcutaneous electrical nerve stimulation, lasertherapy and acupuncture in transformed migraine treatment, Neurol Sci. 24, Suppl 2 (2003): 138-42.
**(8)** Gad Alon; Chapter 3: Principles of electrical stimulation, in Clinical Electrotherapy; Appleton & Lange 1987; pp 29-47.
***(9):*** Instrumentation for Neuromuscular electrical stimulation, Snyder-Mackler L, Robinson AJ, eds.; Clinical Electrophysiology;. Baltimore, Md; Williams & Wilkins; 1989: 99.
**(10):** Instrumentation for Neuromuscular electrical stimulation, Snyder-Mackler L, Robinson AJ, eds.; Clinical Electrophysiology;. Baltimore, Md; Williams & Wilkins; 1989: 100.
***(11):*** Principes Fondamentaux de l'électrostimulation, Medi-Compex 1994 ; Courant optimal ; Durée de l'impulsion électrique rectangulaire, pp. 1.2.3 à 1.2.5.

## Revendications

1. Appareil pour le traitement électrothérapeutique des céphalées, telles que les céphalées de tension et les migraines, comprenant un élément symétrique allongé (10), de support de deux électrodes de contact (11, 12) à appliquer transversalement au niveau de la partie supérieure du visage, dans la région inter-sourciliaire (20), chacune des électrodes (11, 12) étant en contact avec un gel conducteur autocollant appliqué sur la face du support destinée à être appliquée sur la peau du visage, ledit gel conducteur étant appliqué sur deux zones latérales données (13, 14), couvrant essentiellement tout le support (10), à l'exception d'une zone centrale isolante, ainsi qu'un circuit électrique pour l'alimentation desdites électrodes (11, 12) au moyen d'impulsions électriques basse tension, **caractérisé en ce que :**
- le support d'électrode (10) présente une forme et des dimensions choisies pour permettre, de manière indépendante du sujet, l'excitation des afférences des nerfs supratrochléaires (2) et supraorbitaires (3) de la branche ophtalmique (1) du nerf trijumeau ;
- le circuit électrique comprend un générateur de signal programmable apte à créer des impulsions de durée comprise entre 150 et 450 microsecondes avec une montée en intensité de 0 à 20 milliampères maximum à une vitesse inférieure ou égale à 40 microampères par seconde et avec un pas d'augmentation de l'intensité ne dépassant pas 50 microampères.

2. Appareil selon la revendication 1, **caractérisé en ce que** le support d'électrode (10) possède une partie centrale de hauteur H1 supérieure à la hauteur H2 de chacune des parties externes, les extrémités supérieures des deux parties externes étant, une fois le support (10) positionné, à un niveau légèrement inférieur au niveau de la partie centrale.

3. Appareil selon la revendication 1, **caractérisé en ce que** le support d'électrode possède, comme dimensions :
- une longueur ou extension latérale L comprise entre 70 et 115 mm ;
- une hauteur H1 de la partie centrale comprise entre 15 et 50 mm ;
- une hauteur H2 de chacune des parties externes comprise entre 5 et 20 mm.

4. Appareil selon la revendication 3, **caractérisé en ce que :**
- la longueur L a une valeur d'environ 95 mm ;
- la hauteur H1 de la partie centrale a une valeur d'environ 30 mm ;
- la hauteur H2 de chacune des parties externes a une valeur d'environ 10 mm.

5. Appareil selon la revendication 1, **caractérisé en ce que** la durée des impulsions est d'environ 250 microsecondes.

6. Appareil selon la revendication 1, **caractérisé en ce que** le pas d'augmentation de l'intensité est d'environ 30 microampères.

7. Méthode de traitement thérapeutique des céphalées, telles que les céphalées de tension et les migraines, au moyen de l'appareil selon l'une quelconque des revendications 1 à 6, **caractérisé** au moins par les étapes suivantes :
- on place le support d'électrode autocollant (10) sur la partie supérieure du visage dans la région inter-sourciliaire (20) de manière à couvrir, de chaque côté, les afférences du nerf supratrochléaire (2) et supraorbitaire (3) de la branche ophtalmique (1) du nerf trijumeau ;
- on génère, au moyen du générateur de signal programmable, un signal électrique comprenant des impulsions rectangulaires de durée comprise entre 150 et 450 microsecondes, de préférence 250 microsecondes, avec une montée en intensité de 0 à 20 milliampères maximum, à une vitesse inférieure ou égale à 40 microampères par seconde, ladite montée en intensité étant réalisée avec un pas ne dépassant pas 50 microampères, de préférence 30 microampères ;
- on applique ledit signal électrique aux électrodes pour traiter les céphalées de tension et les migraines.
